(19)

**Europäisches Patentamt**
**European Patent Office**
**Office européen des brevets**

(11) **EP 4 494 581 A1**

(12) **EUROPEAN PATENT APPLICATION**
published in accordance with Art. 153(4) EPC

(43) Date of publication:
**22.01.2025 Bulletin 2025/04**

(21) Application number: **23769960.8**

(22) Date of filing: **08.03.2023**

(51) International Patent Classification (IPC):
**A61B 17/56** (2006.01)

(86) International application number:
**PCT/ES2023/070126**

(87) International publication number:
**WO 2023/175210 (21.09.2023 Gazette 2023/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **16.03.2022 ES 202230220**

(71) Applicants:
• **Universidad Complutense De Madrid**
  28040 Madrid (ES)
• **Fundación Para La Investigación Biomédica Del Hospital Gregorio Marñón**
  28028 Madrid (ES)

(72) Inventors:
• **SERRANO LÓPEZ, Dolores Remedios**
  28040 Madrid (ES)

• **YUSTE SOSA, Ivan**
  28040 Madrid (ES)
• **LUCIANO DE LEÓN, Francis Cristina**
  28040 Madrid (ES)
• **BALLESTEROS PAPANTONAKIS, María Paloma**
  28040 Madrid (ES)
• **GONZÁLEZ BURGOS, Elena**
  28040 Madrid (ES)
• **SANZ RUIZ, Pablo**
  28028 Madrid (ES)
• **RIBED SÁNCHEZ, Almudena**
  28028 Madrid (ES)

(74) Representative: **Temiño Ceniceros, Ignacio**
  **Abril Patentes y Marcas, S.L.**
  **Calle Zurbano, 76 - 7°**
  **28010 Madrid (ES)**

(54) **CUSTOMISED IMPLANTS HAVING ANTIMICROBIAL ACTIVITY FOR PREVENTING AND TREATING BONE INFECTIONS**

(57) Current treatments for surgical site infections that are caused by fungi or bacteria with antibiotics and antifungal agents are usually based on the prolonged intravenous administration or use of polymethyl methacrylate cements after removing the prosthesis and cleaning the surrounding tissue, which requires another surgical procedure. However, an administration system that avoids high body exposure to the antimicrobial agent and prevents the patient from having to undergo a new surgery would be more desirable. The present invention discloses 3D printed implants with biocompatible and biodegradable polymers comprising medicinal drugs with immediate-sustained release, having a reduced haemolytic profile, which easily adhere to the prostheses, exhibit a good in vitro efficacy profile against different fungal and bacterial strains and can be sterilised by means of UV light due to the thinness thereof.

**Fig.1**

EP 4 494 581 A1

## Description

## Field of the invention

The present invention falls within the technical field of the manufacture of parenteral administration of drugs with application in orthopedics for the prevention and treatment of bone infections. More specifically, it refers to the design and manufacture of 3D-printed personalized implants loaded with antimicrobial drugs.

## State of the art

Orthopedic surgeries often involve the placement of a foreign body, such as a prosthetic joint, joint components, or others to stabilize bone structures or repair fractures. These implants can promote infection through either direct contamination or the hematogenous spread of microorganisms. Surgically site infections (SSI) account for 30% of all causes of periprosthetic joint infections (PJls).

Currently, the most common treatments used to treat these infections involve the administration of antibiotics for prolonged periods and the use of polymethylmethacrylate cement (PMMA) loaded with antibiotics, after removing the implants and cleaning the surrounding tissues. Cements loaded with antibiotics result, in the first phase, in an early high local concentration of antibiotics that is followed by a second phase in which the drugs are released slowly over several days and even months.

PMMA cement loaded with a single antibiotic (e.g., vancomycin, gentamicin, or ciprofloxacin) has been developed. PMMAs loaded with two antibiotics are currently undergoing clinical trials, with the interaction between drugs remaining an unsolved challenge. On the other hand, to date, there is not commercially available PMMA cement loaded with an antifungal drug, which limits its application in the treatment of fungal infections to this level. The main drawbacks associated with the use of antimicrobials-loaded cements include inadequate drug release from the bone cement post-surgery, particularly for lipophilic drugs, resulting in minimal antibiotic release and consequently increases the risk of microbial resistance. A second limitation is the potential alteration of joint biomechanical properties due to drug incorporation, which can reduce the compressive strength of the cement, compromising the cement strength impacting the patient's quality of life. To overcome these limitations, alternative approaches have been proposed, including the use of taurolidine for bone infection treatment (EP0139534), parenteral administration of antibiotic-coated polymeric particles (US2010/0291220), sustained release of silver ions (US9248254) and *in situ* application of biodegradable microspheres for controlled antibiotic release (US8986737). However, in most cases, there is a problem of bioadhesion to the joint prosthesis, lack of osseointegration and cytotoxicity and poor control in the release of the antibiotic, without existing any dual therapy that acts against bacteria and fungi simultaneously.

For all these reasons, there is still a clinical need for novel strategies to reduce PJls through versatile drug delivery systems with the capacity to contain more than one antibiotic or combination of antibiotics and antifungals to cover a wide spectrum of infections. These systems should not alter the biomechanical properties of the joint and, ideally, should be able to be placed externally on the top of the prosthesis with a millimetric thickness, so they do not hinder the surgical procedure.

## Brief description of the invention

The present invention describes a combined immediate-controlled drug-release implant with antibacterial and antifungal drugs for the treatment of PJls. These formulations represent a solution to the clinical problem derived from the lack of drugs available for the prophylaxis and treatment of PJls caused by exogenous pathogens or those from the patient's skin flora that contaminate implanted prostheses during surgery.

More specifically, a drug delivery system is proposed that facilitates controlled release from an implant designed to mimic a spider web or similar morphology, manufactured via 3D printing with a complementary release profile (immediate and sustained release) aimed at preventing and treating infections, including both acute and delayed-onset scenarios. Amphotericin B (AmB) has been selected as a model antifungal drug, which is currently used as a gold standard for the treatment of systemic fungal infections, and vancomycin as a broad-spectrum antibacterial agent; these implants can be applied directly onto the prostheses during the surgical procedure.

The first aspect of the invention refers to the design and manufacture of parenteral implants in the form of spiderwebs with a morphology adapted to the metallic components of prostheses such as those used in hips, knees, or heart valves, among others. These implants have millimetric dimensions and are composed of biocompatible and biodegradable materials (e.g. polyvinyl alcohol, polyethylene glycol, polycaprolactone or a combination). The design of the network facilitates osseointegration, as it contains spaces of at least 100 $\mu$m. In addition, they have adhesive capacity after wetting in aqueous media and can be adhered to the surface of joint prostheses in minutes. The net allows an immediate release (*burst effect*) combined with a sustained release profile of antimicrobial agents, particularly within the first 24 hours post-implantation, presenting an optimal anti-infective efficacy, both against fungi and bacteria. These nets can also be sterilized using UV light without damaging their structure or composition, due to their thin thickness.

The nets can be manufactured using any 3D printing technique, especially extrusion-based deposition techniques known as FDM (*Fused Deposition Mod-*

*eling*) or by direct powder extrusion. In the first case, filaments previously manufactured by hot melt extrusion with biocompatible polymers are used; Once the network is printed, the spiderweb can be loaded by passive diffusion with anti-infective agents. In the case of direct extrusion, the excipients, and drug in a powdery state are previously mixed and homogenized. Nets can also be manufactured using laser-based printing techniques such as stereolithography and laser sintering.

**Brief description of the drawings**

In order to complement the description that is being made and to help a better understanding of the characteristics of the invention, a set of drawings is attached as an integral part of the description, in which for illustrative and not limited purposes, it has been represented as follows:

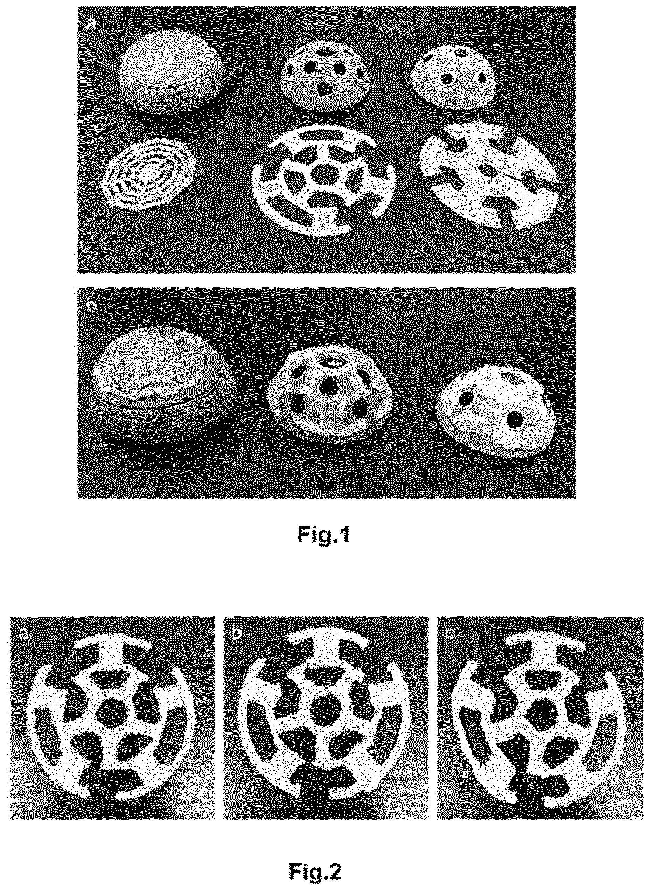

**Figure 1.** Designs of the 3D printed implants in the form of a spiderweb, and their adhesion to the prosthesis: (a) Implants manufactured by FDM adapted to the geometry of different types of acetabular components which are made from polyvinyl alcohol (PVA) partially saponified (> 94.8%) with fragments of polyethylene glycol (PEG); (b) nets adhered to the surface of the acetabular metallic components of a prosthesis after their humectation with water.

**Figure 2.** 3D printed implants obtained by direct powder extrusion consisting of a mixture of polycaprolactone (PCL) and PEG: (a) nets printed with 70% PCL and 30% PEG; (b) nets printed with 80% PCL and 20% PEG; (c) nets printed with 90% PCL and 10% of PEG.

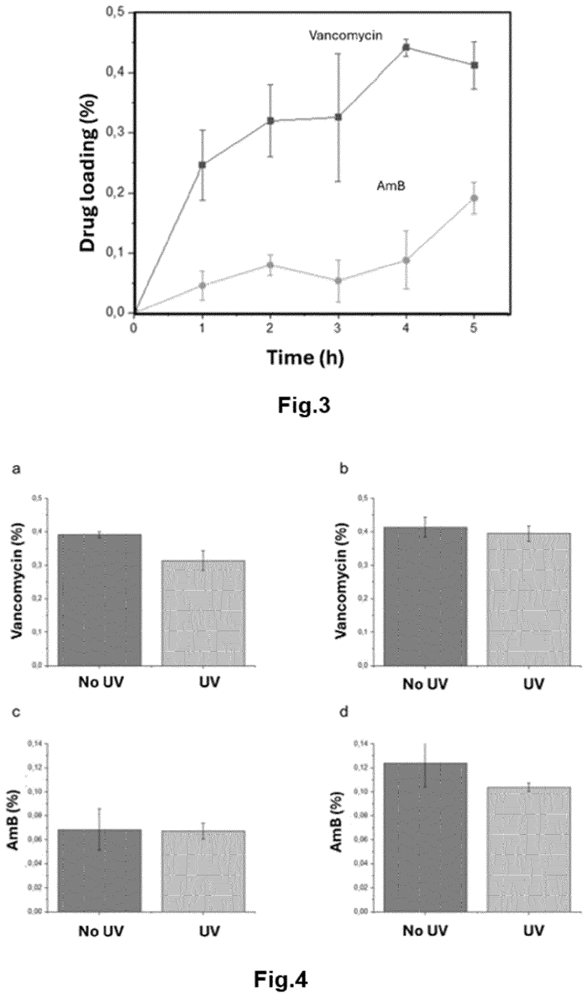

**Figure 3.** Drug loading by passive diffusion with vancomycin and AmB using implants manufactured by FDM.

**Figure 4.** Effect of UV light sterilization on AmB and vancomycin content in a 3D printed implant containing vancomycin, AmB, or both antimicrobials, before exposition to UV light and after 20 minutes of UV exposure. (a) Vancomycin-loaded implants; (b) Vancomycin content in vancomycin- AmB-loaded implants; (c) AmB-loaded implants; (d) AmB content in vancomycin- AmB-loaded implants. Statistically significant differences ($p < 0.05$) are expressed by *.

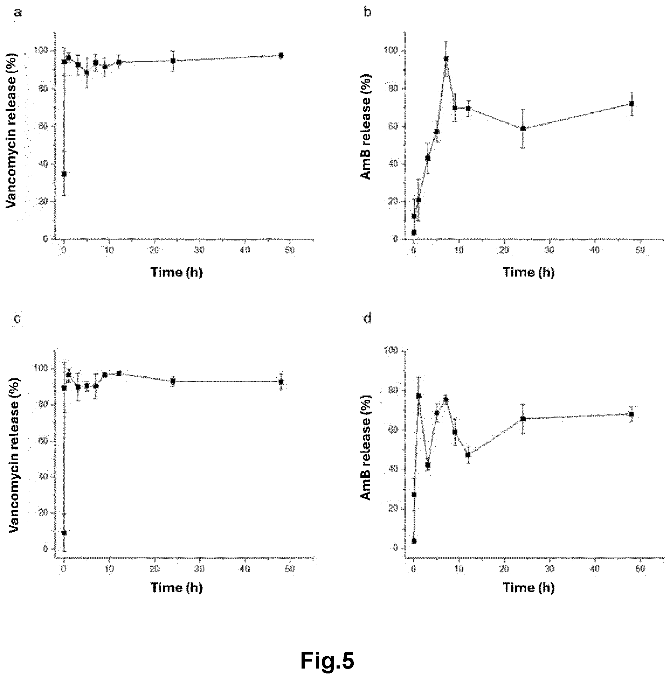

**Figure 5**. Drug release after the adhesion of the implant to the acetabular component of the prosthesis and its subsequent immersion in a physiological medium (PBS, pH = 7, 4): (a) implants loaded only with vancomycin; (b) implants loaded with AmB only; (c) vancomycin released from implants loaded with vancomycin and AmB; (d) AmB released from implants loaded with vancomycin and AmB.

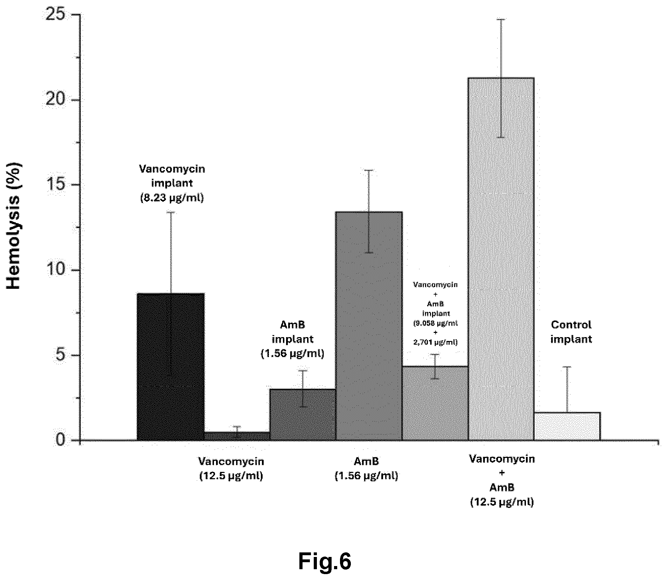

**Figure 6**. Hemolytic toxicity of implants loaded only with vancomycin (Implant V), vancomycin dissolved in DMSO, implants loaded only with AmB (Implant A), AmB dissolved with DMSO, implants loaded with both drugs (Implant V+A), vancomycin and AmB in DMSO, and unloaded implants. The percentage of hemolysis is expressed at the maximum concentration evaluated with each drug in the implants, compared to similar concentrations of active ingredient dissolved in DMSO.

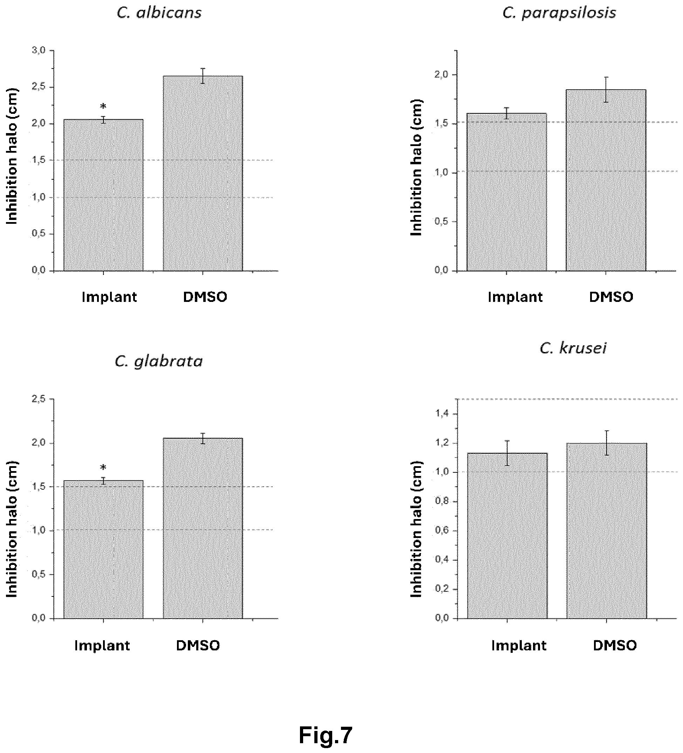

**Figure 7**. In *vitro antifungal* activity of implants loaded with AmB and AmB dissolved in DMSO against different species of *Candida* (*C. albicans, C. glabrata, C. parapsilosis, C. krusei*). Statistically significant differences ($p < 0.5$) are expressed by *. An inhibition halo greater than 1.5 cm indicates that the microorganism is susceptible to the drug; however, a halo smaller than 1 cm in diameter indicates that the microorganism is resistant to the drug according to the CLSI (*Clinical Laboratory Standard Institute*) guidelines. Intermediate inhibition halos indicate that the microorganism is susceptible depending on the dose used.

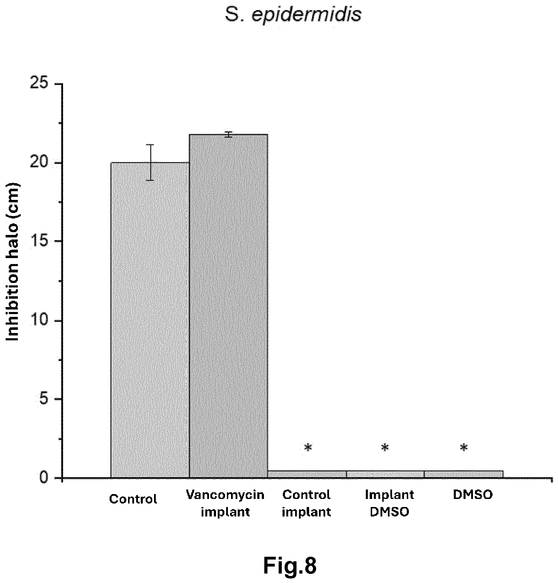

**Figure 8**. In vitro antibacterial activity to inhibit *Staphylococcus epidermis* with commercial vancomycin discs (control, 30 µg), discs loaded with vancomycin implant, unloaded implant (control), implant impregnated with DMSO and pure DMSO used as 100% control. Statistically significant differences ($p < 0.5$) are expressed by*. An inhibition halo greater than 1.5 cm indicates that the microorganism is susceptible to the drug according to CLSI guidelines.

**Detailed description of the invention**

The present invention is illustrated by the following examples, which are not intended to be limited to its scope.

**Example 1**. This example refers to the design and 3D printing of implants from a filament using the FDM method (Figure 1).

First, the measurements of different acetabular components were recorded so, subsequently, a design that fits the surface was created using a CAD (*Computer Aided Design*) program, such as *Tinkercad* or *Solid Works.* In the case of acetabular components with a full solid surface, a spider web-like structure was designed with several arms that allow better adhesion to the cup and with a certain separation between the fibers, at least 100 µm, to ensure osseointegration.

Any FDM printer can be used to 3D print the implants. In this example, a *Hyrel 3D Engine* SR printer (Georgia, USA) was used, with an extrusion nozzle of 0.5 mm diameter; a filament composed of polyvinyl alcohol (PVA) partially saponified (>94.8%) with fragments of polyethylene glycol (PEG) was used. The temperature of the extruder (MK1-250) oscillated in the range of 210-215°C and the temperature of the platform was maintained at 70°C to ensure good adhesion of the 3D design.

The height of the implant was optimized at 1 mm to increase the drug loading by passive diffusion.

Figure 1 shows how the designed and printed net fits perfectly to the dimensions of the acetabular component, indicating that it can be customized for any prosthesis dimension.

**Example 2**. This example refers to the adhesion of the implants to their respective acetabular cups (Figure 1).

To attach the PVA and PEG implants to the cup described in example 1, these were immersed for a few seconds in deionized water until they acquired a sticky texture. Subsequently, they were placed on the cup exerting a slight pressure (making them adapt to the convex surface of the cup) and maintaining such pressure until the implant was completely adhered to. This highlights the adhesive nature of implants, which can adhere to the surface of the cup within minutes. This characteristic is particularly advantageous when there is a need for rapid implantation during surgical operations.

**Example 3**. This example refers to the 3D printed design of implants obtained from a mixture of powdery polymers using direct powder extrusion 3D printing technology (Figure 2).

Ten grams of a combination of PCL and PEG were prepared with different weight ratios (90:10, 80:20, and 70:30). The mixture of the two polymers was micronized with a mill until a fine and homogeneous powder was obtained that was sieved before printing.

A Hyrel 3D Engine SR printer (Georgia, USA) and a KR2-15 extruder with a 0.5 mm aperture extrusion nozzle were used to print the implants. This extruder had a hollow metallic barrel where the powdery material was loaded. Once the barrel was filled with the polymer mixture, it was coupled to the printer and heated to 75°C. The temperature of the bed was 55°C. Under these conditions, the flow of molten polymer through the extruder nozzle allowed the 3D implants to be printed with high resolution.

**Example 4.** This example refers to the loading of implants made of PVA-PEG with 0.4% vancomycin and 0.1% AmB by passive diffusion (Figure 3).

One hundred and fifty milligrams of the active substance (vancomycin or AmB) were dissolved in 7.5 ml of DMSO, followed by vortex and sonication for 15 minutes. In a Petri dish, 22.5 ml of ethanol was added and mixed slowly with the DMSO solution with the active ingredient dissolved to avoid precipitation.

The printed implants are then immersed in the liquid mixture. The plate was covered and sealed with parafilm to prevent evaporation of the solvent. At certain times, a fragment of the implant was taken and dissolved in 10 ml of distilled water with the help of a vortex and sonicator and the drug content was analyzed by HPLC to determine the amount of active ingredient that was diffused within the implant.

The HPLC was equipped with a *Jasco PU-1580 pump,* a Jasco AS-2050 Plus *automatic injector and* a Jasco UV-1575 *UV-visible detector.* The integration of the peaks was done with the Borwin 1.5 *program* for PC (*JMBS Developments*). The AmB was separated into a *Thermo Hypersil-Keystone BDS* column (200 × 4.4 mm, 5um). The mobile phase consisted of acetonitrile: acetic acid: distilled water (52:4,3:43,7, V:V:V), which was pumped at a flow rate of 1 mL/min and the sample injection volume was 40 μL. The column temperature was maintained at 25°C and the detector was set to 406 nm. In the case of vancomycin, a *Nucleosyl* C18 column (250 × 4.6 mm, 5um) was used. The mobile phase consisted of ammonium phosphate 50mM: acetonitrile (92:8, V:V), with a final pH of 2.2, which was pumped at a flow rate of 0.7 mL/min and the sample injection volume was 40 μL. The column temperature was maintained at 25°C and the detector was set to 205 nm. Before the experiment, a triplicate calibration curve was performed for AmB and vancomycin to determine the linearity, specificity and the detection limit of the method.

For vancomycin, passive diffusion loading kinetics were linear at a rate of 0.096%/h ($R^2$ = 0.8545) up to 4 hours. Longer times did not result in greater drug loading.

In the case of AmB, the loading kinetics were bilinear, with a kinetic of 0.0042%/h up to 3 hours and 0.047%/h from 3 to 6 hours. At shorter times, the geometry of the implant was still very rigid, but, after three hours of wetting, the network became more flexible allowing a greaterAmB loading.

Compared to vancomycin, the loading process of AmB was slower due to its low solubility in aqueous medium and its lower miscibility with the materials used to print the implant.

The drug loading process can also be carried out using the direct powder extrusion technique for which the drugs were mixed with the polymers to form a powdery mixture and then extruded under the conditions illustrated in example 3.

Example 5. This example refers to the sterilization of manufactured implants using UV light with PVA-PEG and its effect on AmB and vancomycin content (Figure 4).

Implants loaded with different active ingredients (vancomycin and/or AmB) were dried in the open air for 24 hours, stored in a transparent airtight bag, and placed under UV light for 20 minutes. After that time, the implants were turned over and exposed to UV light for an additional 10 minutes. Once the sterilization process was completed, the implants were collected, cut in small fragments and the drug content was compared before and after exposure to UV light. To determine the amount of active ingredient in the implants, HPLC was used, as described in example 4.

Although in all cases a slight decrease in drug content was observed, no statistically significant differences (p > 0.05) before and after UV light sterilization were found. The vancomycin and AmB content in the implant remained the same at 0.4% and 0.1% respectively.

Microbial growth was not observed in any of the agar plates after placing a small fragment of the implant exposed to the UV light for 20 min. Therefore, it was concluded that the UV sterilization method was sufficient to ensure the microbiological quality of the implants and did not affect the drug content.

**Example 6.** This example refers to the release of the active ingredients from the PVA-PEG 3D printed implant after its adhesion to the acetabular cup and its subsequent immersion in a physiological medium (PBS pH 7.4) with albumin (Figure 5).

Implants loaded with vancomycin, AmB and both active ingredients were immersed for a few seconds in deionized water to enhance their adhesive character and then were adhered to the cups. Afterward, the prosthesis with the 3D implant attached to its surface was placed face down on a concave receptacle designed in 3D with a size similar to the cup imitating the joint space, with a capacity of 10 ml. The physiological medium consisting of 10 mL of PBS pH 7.4 with 20% albumin (80:20, V:V) was added to mimic physiological conditions (the addition of albumin to the medium was key considering the high plasma protein binding of AmB). The receptacles were covered with *parafilm* to prevent evaporation of the medium and were maintained at 37°C for 48 h. At different times, an aliquot was taken from the medium (750 $\mu$l) and diluted with methanol (1/1, V/V). The receptacle volume was replaced with 750 $\mu$L of additional PBS pH 7.4 with albumin, to maintain *sink conditions.*

The diluted samples were frozen for 24 hours to precipitate the proteins from the medium before HPLC analysis. The samples were then centrifuged at 10,000 rpm for 15 minutes. The supernatant (350 $\mu$l) was diluted (1:3, V/V) with methanol (1,050 $\mu$l) and centrifuged (10,000 rpm for 15 min). The supernatant was analyzed by HPLC to quantify the drug content released from the 3D implants at different times.

In the case of vancomycin, it was observed that both implants loaded with either just vancomycin or vancomycin-AmB showed an immediate release of practically 100% during the first hour. These concentrations remained stable over time for 48 hours.

In the case of AmB, the release was sustained for 10 hours. Afterwards, AmB levels decreased, probably due to a *spring-and-parachute* effect as a result of the amorphous drug gradually recrystallizing in the aqueous medium, decreasing its solubility and, therefore, limiting its concentration) associated with the lower solubility of AmB in aqueous medium compared to vancomycin.

Nevertheless, in both cases, the concentration maintained in the medium after the drug was released from the implant was around 200 $\mu$g/ml for AmB and 400 $\mu$g/ml for vancomycin which was above the minimum inhibitory concentration, indicating that the loaded drug in the implants was suitable to exert the desired antimicrobial effect.

**Example 7**. This example shows the hemolytic effect of PVA-PEG implants loaded with the different active ingredients (AMB and vancomycin), the unloaded implants and the active ingredients dissolved in DMSO separately and in combination (Figure 6).

Red blood cells were obtained by centrifugation of blood from healthy volunteers ($5.48 \times 10^6$ RBC/ml) contained in Vacutainer tubes, coated with EDTA, for 5 min at 1000 rpm. The supernatant (the plasma) was discarded and the erythrocytes were washed three consecutive times with an equivalent volume of 0.9% NaCl (150 mN) centrifuged at each step at 1000 rpm for 5 min. After washing, erythrocytes (RBCs) were diluted in PBS pH 7.4 to a final concentration of 4%. A volume of 180 $\mu$L was then added to each well of a 96-well plate.

To test the effect of the implants and the active ingredients, different serial solutions were prepared, in deionized water and DMSO, respectively. 20 $\mu$l of each concentration (ranging from 50 to 0.01 $\mu$g/mL) was added in each well. A 20% solution of Triton X was used as a positive control, and PBS pH 7.4 was used as a negative control. The plates were incubated at 37°C for 1 hour. They were then centrifuged at 2500 rpm for 5 minutes to sediment the intact erythrocytes. The supernatant (100 $\mu$L) was transferred to a clear, flat-bottomed 96-well plate. Absorbance (ABS) was measured using a plate reader (*BioTeK, EKx808*) at 595 nm. The percentage of hemolysis was calculated using the following equation:

$$Hemolysis\ (\%) = \frac{(ABS\ sample - ABS\ PBS)}{(ABS\ triton - ABS\ PBS)}$$

In the case of the implants, only the highest concentration evaluated was the one that showed any sign of toxicity, being in all cases below 10%. The reduction of the hemolytic toxicity observed between the AmB implant (toxicity less than 5%) compared to the drug dissolved in DMSO was significantly different. Similarly, a significant reduction in toxicity (almost five times lower) was obtained with the combined implant of AmB and vancomycin compared to the combination of both active ingredients dissolved in DMSO at the same concentration. This may be due to the sustained release of the drug from the implant, resulting in minimal hemolytic toxicity. In the case of vancomycin, this effect was not relevant since it is not a hemolytic drug.

**Example 8.** This example demonstrates the *in vitro* antifungal activity of PVA-PEG implants loaded with 0.1% AmB (Figure 7). The antifungal activity of AmB-loaded implants against four different strains of *Candida spp.* (*Candida spp., Candida albicans*, CECT 1394, *Candida parassilopsis* 57744, *Candida glabrata* 60750, and *Candida krusei* 1068) was evaluated by agar diffusion assay (Ruiz, H.K. et al. Int, J. Pharm, 2014. 473(1-2): p. 148-57). To fit the implants in the petri dishes, 13 mm implants in size were printed and placed in the center of the plates on the surface of the agar. As a positive control, discs loaded with AmB dissolved in DMSO (10 μg) were used.

The diameters of the inhibition zone were measured at the points where there was a complete inhibition of yeast growth. The isolates are classified according to the CLSI as susceptible (S) to AmB when the inhibition zone is greater than 15mm; resistant (R), if the inhibition zone is higher than 10 mm; and intermediate (I) if the inhibition zone is between 11 and 14 mm.

Figure 7 shows that the C. *albicans, C. parapsilosis* and *C. glabrata* were susceptible to implants loaded with AmB, with an inhibition halo greater than 15 mm, indicating that there was good diffusion of the drug from the implant to the agar. On the other hand, in the case of *C. krusei,* both implants and AmB in DMSO were in the intermediate zone, so effectiveness against this strain was dose-dependent. These results were promising considering that C. *krusei* is one of the most resistant strains to AmB and even so, the efficacy was dose-dependent.

**Example 9**. This example shows the in vitro antimicrobial activity of PVA-PEG implants loaded with 0.4% vancomycin.

The antibacterial activity of vancomycin-loaded implants was tested and compared with the values obtained with the active ingredient against a strain of *Staphylococcus epidermidis,* ATCC 12228. Antimicrobial activity was tested by the diffusion assay on Kirby-Bauer agar (Ursulino Pontes, E. K. et al. Food Sci Biotechnol, 2019. 28(3): p. 633-639.). For the implants to fit in the Petri dishes, 13 mm in diameter implants were printed, loaded according to the methodology described in example 8 and placed in the center of the plates on their surface.

The diameters of the inhibition zone were measured at points where there was complete inhibition growth of bacteria after 24 h of incubation. Isolates are classified as susceptible to vancomycin (S) when the inhibition zone is greater than 15 mm according to CLSI guidelines.

Figure 8 shows that *S. epidermidis* was susceptible to the vancomycin-loaded implant, with an inhibition halo greater than 15 mm. In addition, there were no significant differences compared to the commercial Neo-Sensitabs vancomycin discs™ loaded with 30 μg of drug (p>0.05). On the other hand, unloaded implants, DMSO, and DMSO-impregnated implants showed no inhibition halo indicating that antibacterial activity occurs only due to the release of vancomycin from the 3D-printed implant.

**Claims**

1. Parenteral implant with an immediate-controlled drug release, consisting of biocompatible and biodegradable materials with a spiderweb shape of a millimetric thickness and contains anti-infective active ingredients.

2. Implant, according to claim 1, where the biocompatible materials of the implant are polyvinyl alcohol, polycaprolactone, polyethylene glycol or a mixture thereof.

3. Implant, according to claim 1, where the spiderweb consists of a central circle with multiple radial filaments with several central supports.

4. Implant, according to claim 3, where the radial filaments can be joint or separated with a gap.

5. Implant, according to claim 4, where the gaps are at least of 100 μm.

6. Implants, according to previous claims, where filaments have the following dimensions ranging from 10-30 mm length and 5-10 mm width.

7. Implants, according to previous claims, where the active ingredient is an antibiotic.

8. Implant, according to claim 5, where the antibiotic is vancomycin.

9. Implant, according to claims 1 to 6, where the active ingredient is an antifungal.

10. Implant, according to claim 9, where the antifungal is amphotericin B.

11. Implant, according to claims 1 to 6, characterized for containing both antibiotics and antifungals.

12. Implant, according to claim 11, where the antibiotic is vancomycin, and the antifungal is amphotericin.

13. Method of obtaining the claimed implant comprising:

   - Design the shape of the implant.
   - Print the implant designed using a 3D printing technique with biocompatible and biodegradable materials.
   - Load the printed implant with anti-infective active ingredients through passive diffusion.

14. Method, according to claim 13, where the implant is designed based on the dimensions of the acetabular cup on which the implant means to be fitted using a CAD (Computer Aided Design) program.

**15.** Method, according to claim 13, where the 3D printing implant is made from a biocompatible or biodegradable filament using FDM (Fused Deposition Modeling).

**16.** Method, according to claim 13, where the 3D printing implant is made from a biocompatible or biodegradable filament using a direct powder extrusion method.

**17.** Method, according to claim 13, where the biocompatible materials that constitute the implant are polyvinyl alcohol, polycaprolactone, polyethylene glycol or a mixture of them.

**18.** Method, according to claim 13, where the active anti-infective ingredients are antibiotics, antifungals or a mixture of them.

**19.** Method, according to claim 18, where the anti-infective active ingredients are vancomycin, amphotericin B, or a mixture of them.

**20.** Method, according to claims 13 to 19, where, optionally, the implant obtained is sterilized by UV light.

**21.** Use of claimed implants can be for the prevention and treatment of infections derived from arthroplasties or heart valvopathies.

**22.** Use of claimed implants, according to claim 20, where the implants is previously wetted before administration directly during the surgical procedure.

**Fig.1**

**Fig.2**

**Fig.3**

**Fig.4**

Fig.5

**Fig.6**

**Fig.7**

S. *epidermidis*

Fig.8

| | INTERNATIONAL SEARCH REPORT | International application No. |
|---|---|---|
| | | PCT/ES2023/070126 |

**A. CLASSIFICATION OF SUBJECT MATTER**

*A61B17/56* (2006.01)

According to International Patent Classification (IPC) or to both national classification and IPC

**B. FIELDS SEARCHED**

Minimum documentation searched (classification system followed by classification symbols)
A61B

Documentation searched other than minimum documentation to the extent that such documents are included in the fields searched

Electronic data base consulted during the international search (name of data base and, where practicable, search terms used)

EPODOC, INVENES, WPI, BIOSIS, MEDLINE, EMBASE, INSPEX, COMPDX

**C. DOCUMENTS CONSIDERED TO BE RELEVANT**

| Category* | Citation of document, with indication, where appropriate, of the relevant passages | Relevant to claim No. |
|---|---|---|
| X | CORDUAS F *et al*. Bio-design And Manufacturing 2021 Springer Germany., 30/11/2020, Vol. 4, N° 2, pages 278 - 310 [on line][retrieved the 1/07/2022]. ISSN 2096-5524 (print), <DOI: 10.1007/s42242-020-00108-1> Pages 280, 290-292 | 1-12 y 21-22 |
| A | GARCIA-GARCIA C *et al*. Journal Of The Mechanical Behavior Of Biomedical Materials Netherlands 01 2022., 31/12/2021, Vol. 125, pages 104909 [on line][retrieved on 01/01/2022]. ISSN 1878-0180 (Electronic), <DOI: 10.1016/j.jmbbm.2021.104909 pubmed:34736025> figure 1 | 1-12 |

☒ Further documents are listed in the continuation of Box C.　　☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | "&" document member of the same patent family |

| Date of the actual completion of the international search | Date of mailing of the international search report |
|---|---|
| 05/06/2023 | **(16/06/2023)** |

| Name and mailing address of the ISA/ | Authorized officer |
|---|---|
| OFICINA ESPAÑOLA DE PATENTES Y MARCAS Paseo de la Castellana, 75 - 28071 Madrid (España) Facsimile No.: 91 349 53 04 | C. González Valdespino Telephone No. 91 3498541 |

Form PCT/ISA/210 (second sheet) (July 2022)

## INTERNATIONAL SEARCH REPORT

| | International application No. |
|---|---|
| | PCT/ES2023/070126 |

C (continuation).                                        DOCUMENTS CONSIDERED TO BE RELEVANT

| Category * | Citation of documents, with indication, where appropriate, of the relevant passages | Relevant to claim No. |
|---|---|---|
| X | DOMSTA VANESSA *et al.* Molecules (basel, Switzerland) Switzerland 02 Jul 202., 02/07/2021, Vol. 26, N° 13 [on line][retrieved on 01/07/2022]. ISSN 1420-3049 (Electronic), <DOI: 10.3390/molecules26134066 pubmed:34279405> Abstract, figure 1. | 13-20 |
| A | SUHARDI V J *et al.* Nature Biomedical Engineering, 20170601 Nature Pub. Group, 01/06/2017, Vol. 1, N° 6, pages 1 [on line][retrieved on 01/07/2022]. ISSN 2157-8451, <DOI: 10.1038/s41551-017-0080> Page 6 | 1-12 |
| A | TIWARI ARJUN PRASAD *et al.* Bimodal fibrous structures for tissue engineering: Fabrication, characterization and *in vitro* biocompatibility. Page 2 | 13-20 |
| A | WO 2019157142 A1 (UNIV CORNELL) 15/08/2019, Figures 1a-1c; Paragraph [0092] | 1-12 |

Form PCT/ISA/210 (continuation of second sheet) (July 2022)

## INTERNATIONAL SEARCH REPORT

Information on patent family members

International application No.

PCT/ES2023/070126

| Patent document cited in the search report | Publication date | Patent family member(s) | Publication date |
|---|---|---|---|
| WO2019157142 A1 | 15.08.2019 | US2020390823 A1 | 17.12.2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0139534 A
- US 20100291220 A
- US 9248254 B
- US 8986737 B

**Non-patent literature cited in the description**

- **RUIZ, H.K. et al.** *Int, J. Pharm*, 2014, vol. 473 (1-2), 148-57
- **URSULINO PONTES, E. K. et al.** *Food Sci Biotechnol*, 2019, vol. 28 (3), 633-639